# EUROPEAN PATENT APPLICATION

(11) **EP 4 803 607 A1**
(43) Date of publication of application: **09.09.2026**
(21) Application number: 24900350.0
(22) Date of filing: 05.11.2024
(51) Int. Cl.: C12M 3/00, C12M 1/00, C12M 1/26

(54) **CULTURE VESSEL AND METHOD FOR PRODUCING CULTURE VESSEL**

(30) Priority: 06.12.2023 JP 2023206456; 07.12.2023 JP 2023207161
(71) Applicant: FUJIFILM Corporation, Tokyo 106-8620 (JP)
(72) Inventor: KASAI, Yusuke, Ashigarakami-gun, Kanagawa 258-8577 (JP); WAKABAYASHI, Akira, Ashigarakami-gun, Kanagawa 258-8577 (JP); SHIGESADA, Keiji, Ashigarakami-gun, Kanagawa 258-8577 (JP); OBA, Takahiro, Ashigarakami-gun, Kanagawa 258-8577 (JP)
(74) Representative: Hoffmann Eitle
(86) International application number: PCT/JP2024/039306
(87) International publication number: WO 2025/121055

(57) **Abstract**

A culture vessel includes: a main body portion that is formed in a tubular shape and that has an opening at an upper end portion; and a bottom member that is fixed to a lower end portion of the main body portion to constitute a bottom of the main body portion, and that is formed with a culture surface on which cells are culturable, in which the bottom member is formed of a degradable material.

## Description

### BACKGROUND OF THE INVENTION

### 1. Field of the Invention

The technology of the present disclosure relates to a culture vessel and a manufacturing method of a culture vessel.

### 2. Description of the Related Art

JP2011-517696A discloses a non-biodegradable membrane for supporting cell proliferation. According to this document, a membrane on which cells are cultured is cut with a scalpel in a tissue culture flask, and the cells are recovered. In addition, JP2005-348736A discloses a support device for a cell culture membrane for culturing corneal cells. According to this document, the cell culture membrane on which corneal cells are cultured is held by being sandwiched between upper and lower members.

### SUMMARY OF THE INVENTION

In the technology disclosed in JP2011-517696A, since cells are cultured on a non-biodegradable membrane, the application is limited. On the other hand, in the technology disclosed in JP2005-348736A, a biodegradable substrate is used for the cell culture membrane, and, for example, it is possible to degrade the substrate in a living body.

Here, in a case of collecting a cell piece cultured on a membrane body or the like, it is required to simplify the work as much as possible. However, in the technology of JP2011-517696A, since the membrane is cut with a scalpel, technical skill of an operator is required. In addition, although JP2005-348736A discloses that a cornea having a desired size is collected by punching, since the cell culture membrane is held by being sandwiched between the upper and lower members, there is a possibility that, in collecting the cornea, time and effort is required to disassemble the upper and lower members and remove the cell culture membrane.

The technology according to the present disclosure provides a culture vessel and a culture method capable of reducing time and effort in a case of collecting cells.

A culture vessel according to a first aspect includes a main body portion that is formed in a tubular shape and that has an opening at an upper end portion, and a bottom member that is fixed to a lower end portion of the main body portion to constitute a bottom of the main body portion and that is formed with a culture surface on which cells are culturable, in which the bottom member is formed of a degradable material.

In the culture vessel according to the first aspect, cells can be cultured on the bottom member. The bottom member is fixed to the lower end portion of the main body portion to constitute the bottom of the main body portion. Therefore, in a case where the culture vessel is placed on a pedestal or the like, a cell collection instrument is inserted from the upper end portion of the tubular main body portion, and a distal end of the cell collection instrument is pressed against the bottom member, a reaction force is obtained from the pedestal, and the cultured cells can be punched out and collected together with the bottom member.

As described above, according to the culture vessel, it is possible to omit the time and effort of disassembling the members in a case of collecting the cells, as compared with a configuration in which the cell culture membrane is sandwiched and fixed between a plurality of members from above and below. That is, it is possible to reduce the time and effort in collecting the cells. In addition, since the number of members can be reduced, there is little risk of contamination.

Further, the bottom member is formed of a degradable material.

As a result, after collecting the cells together with the bottom member by a cell collection instrument, in a case where the bottom member is degraded, it is possible to use only the cells (for example, for transplantation into a living body).

A culture vessel according to a second aspect is the culture vessel according to the first aspect, in which the bottom member is adhered to a lower end surface of the main body portion.

In the culture vessel according to the second aspect, the bottom member is formed as a separate member from the main body portion and is adhered to the main body portion. Therefore, it is possible to select a material according to the performance required for the bottom member.

A culture vessel according to a third aspect is the culture vessel according to the second aspect, in which the bottom member is thermally fusion-welded to the lower end surface.

In the culture vessel according to the third aspect, the bottom member is thermally fusion-welded to the main body portion. Therefore, it is not necessary to use a material such as an adhesive.

A culture vessel according to a fourth aspect is the culture vessel according to the third aspect, in which the bottom member is thermally fusion-welded to an outer portion of the lower end surface from a position offset outward from the culture surface.

In the culture vessel according to the fourth aspect, the bottom member is thermally fusion-welded to an outer portion of the lower end surface of the main body portion from a position offset outward from the culture surface. Therefore, it is possible to reduce the influence of heat on the culture surface as compared with a case where no offset is provided.

A culture vessel according to a fifth aspect is the culture vessel according to the third aspect, in which a surface free energy of the main body portion is -10 mJ/m² or more and +10 mJ/m² or less with respect to a surface free energy of the bottom member.

According to the culture vessel according to the fifth aspect, the bondability by thermal fusion-welding is improved as compared with a case where the surface free energy of the main body portion is smaller than -10 mJ/m² or larger than +10 mJ/m² with respect to the surface free energy of the bottom member.

A culture vessel according to a sixth aspect is the culture vessel according to the first aspect further comprising a support member that includes a support portion having higher rigidity than the bottom member and a holding portion that holds the support portion below the bottom member.

The culture vessel according to the sixth aspect comprises the support member, and the support portion having high rigidity is disposed below the bottom member. As a result, deflection of the bottom member can be reduced.

A culture vessel according to a seventh aspect is the culture vessel according to the sixth aspect, in which the holding portion is fitted to the main body portion to hold the support portion.

In the culture vessel according to the seventh aspect, the holding portion of the support member is fitted to the main body portion. Therefore, the support portion can be held below the bottom member without relying on other members.

A culture vessel according to an eighth aspect is the culture vessel according to the sixth aspect, in which the holding portion is a pedestal that supports the support portion from below.

In the culture vessel according to the eighth aspect, the holding portion of the support member is a pedestal that supports the support portion from below. Therefore, the support portion can be held below the bottom member with a simple configuration as compared with a case where the holding portion of the support member is fitted to the main body portion.

A culture vessel according to a ninth aspect is the culture vessel according to the first aspect, in which the bottom member is a porous body.

In the culture vessel according to the ninth aspect, the bottom member is a porous body. Therefore, by immersing the culture vessel in a culture solution, the bottom member is infused with the culture solution, and the bottom member is immersed in the culture solution. Therefore, the culture solution is supplied to the bottom member from an up-down direction. As a result, the culture efficiency of the cells is improved. On the other hand, in a case where the bottom member is not porous, the culture solution is supplied only from one side of the bottom member, and thus the culture efficiency is relatively poor.

A culture vessel according to a tenth aspect is the culture vessel according to the first aspect, in which the bottom member is formed of a fibrous material.

In the culture vessel according to the tenth aspect, the bottom member is formed of a fibrous material. Therefore, by immersing the culture vessel in a culture solution, the bottom member is infused with the culture solution, and the bottom member is immersed in the culture solution. Therefore, the culture solution is supplied to the bottom member from an up-down direction. As a result, the culture efficiency of the cells is improved. On the other hand, in a case where the bottom member is not porous, the culture solution is supplied only from one side of the bottom member, and thus the culture efficiency is relatively poor.

A culture vessel according to an eleventh aspect is the culture vessel according to the ninth aspect or the tenth aspect, in which the culture vessel further comprises a support member that includes a support portion having higher rigidity than the bottom member, and a holding portion that holds the support portion below the bottom member, and the support portion is formed of a porous body.

In the culture vessel according to the eleventh aspect, the support portion of the support member is formed of a porous body. Therefore, in a case where the culture solution is supplied from the lower side of the bottom member, the support portion is infused with the culture solution. Therefore, the supply of the culture solution to the bottom member is less likely to be hindered by the support portion.

A culture vessel according to a twelfth aspect is the culture vessel according to the first aspect, in which the bottom member is a material that is degraded by biodegradation.

In the culture vessel according to the twelfth aspect, the bottom member is degraded by biodegradation. As a result, after collecting the cells together with the bottom member by an access instrument such as a cell collection instrument, even in a case where the cells are transplanted into a living body together with the bottom member, the bottom member is degraded in the living body, and only the cells are transplanted.

A culture vessel according to a thirteenth aspect is the culture vessel according to the first aspect, in which an outer peripheral surface of an access instrument for accessing the culture surface, the access instrument being inserted from the opening at the upper end portion, comes into contact with at least a part of an inner peripheral surface of the main body portion so as to guide a distal end of the access instrument to a predetermined position on the culture surface.

In the culture vessel according to the thirteenth aspect, cells can be cultured on the bottom member at the lower end portion of the main body portion. Then, the access instrument for accessing the culture surface can be brought close to the cultured cells. For example, in a case where a cell collection instrument is used as the access instrument, it is possible to collect the cells by the cell collection instrument that is inserted from the upper end portion of the tubular main body portion. In this case, the outer peripheral surface of the access instrument comes into contact with at least a part of the inner peripheral surface of the culture vessel and the distal end of the access instrument is guided to a predetermined position on the culture surface.

That is, an access range of the access instrument to the culture surface is positioned. In a case where a cell acquisition instrument is used as the access instrument, a cell collection range is positioned. As a result, as compared with a configuration in which the distal end of the access instrument is not guided to a predetermined position on the culture surface, a cell collection operation can be easily performed, and operator dependency and operational errors are reduced. In addition, the cell collection range to the culture surface is relatively increased. As described above, the yield of cells can be increased.

On the other hand, in a case where the distal end of the access instrument is not guided to a predetermined position on the culture surface and the access range is not positioned, for example, the cell collection position is not determined, so that a deviation may occur during cell collection, which may affect the integrity of cell tissue. Therefore, the cell collection operation is difficult. In addition, by the amount that a movable range of the distal end of the cell collection instrument is secured on the culture surface, the cell collection range to the culture surface is relatively narrowed, and the yield of cells is reduced.

A culture vessel according to a fourteenth aspect is the culture vessel according to the thirteenth aspect, in which the culture surface is, at least in part, offset outward at equal intervals from the distal end of the access instrument.

In the culture vessel according to the fourteenth aspect, the culture surface is, at least in part, offset outward at equal intervals from the distal end of the access instrument. For example, in a case where the distal end of the access instrument has an oval shape, the culture surface has a portion offset outward at equal intervals along the oval shape. As a result, the yield of cells can be increased.

On the other hand, in a case where the culture surface is not offset outward at equal intervals from the distal end of the access instrument, for example, in a case where the distal end of the access instrument has an oval shape and the culture surface has a circular shape, a region where cells cannot be collected is increased in a short-side direction of the oval. Therefore, it is difficult to increase the yield of cells.

A culture vessel according to a fifteenth aspect is the culture vessel according to the thirteenth aspect, in which, in a state in which the outer peripheral surface is in contact with the inner peripheral surface, a minimum distance between an edge of the culture surface and the distal end of the access instrument is more than 0.2% and less than 20% with respect to a maximum dimension between the edges.

According to the culture vessel of the fifteenth aspect, the minimum distance between the edge of the culture surface and the distal end of the access instrument is more than 0.2% with respect to the maximum dimension between the edges. Therefore, as compared with a case of 0.2% or less, a clearance around the distal end of the access instrument is widened, and thus manufacturing tolerances of the culture vessel and the cell collection instrument are easily absorbed.

In addition, according to the culture vessel, the minimum distance between the edge of the culture surface and the distal end of the access instrument is less than 20% with respect to the maximum dimension between the edges. Therefore, as compared with a case of 20% or more, the clearance around the distal end of the access instrument is narrowed, and thus the yield of cells can be increased.

A culture vessel according to a sixteenth aspect is the culture vessel according to the thirteenth aspect, in which contact portions with which the outer peripheral surface is configured to come into contact are formed at a plurality of locations on the inner peripheral surface, and each of the contact portions guides the distal end to a different position on the culture surface.

According to the culture vessel of the sixteenth aspect, a plurality of cell pieces can be collected from one culture surface.

A culture vessel according to a seventeenth aspect is the culture vessel according to the thirteenth aspect, in which a shape of the culture surface is non-circular.

According to the culture vessel of the seventeenth aspect, it is easy to collect a non-circular cell piece.

A culture vessel according to an eighteenth aspect is the culture vessel according to the thirteenth aspect, in which a cross-sectional area of a portion surrounded by the inner peripheral surface gradually decreases from the opening at the upper end portion toward the culture surface.

According to the culture vessel of the eighteenth aspect, it is easy to guide the access instrument that is inserted from the opening at the upper end portion to the culture surface.

A manufacturing method of a culture vessel according to a nineteenth aspect, the manufacturing method including: a step of disposing, on a lower end surface of a main body portion that is formed in a tubular shape and that has openings at an upper end portion and a lower end portion, a bottom member that is formed with a culture surface on which cells are culturable and that is formed of a degradable material, in a state in which the opening is covered; and a step of heating the bottom member with a heat source that is disposed with a buffer member interposed between the bottom member and the heat source, and thermally fusion-welding the bottom member to the lower end surface, in which the buffer member has non-adhesiveness such that the bottom member does not adhere to the buffer member at a melting temperature of the bottom member.

In the manufacturing method of a culture vessel according to the nineteenth aspect, the bottom member is fixed to the lower end surface of the main body portion. Therefore, in a case where the culture vessel is placed on a pedestal or the like, a cell collection instrument is inserted from the upper end portion of the tubular main body portion, and a distal end of the cell collection instrument is pressed against the bottom member, a reaction force is obtained from the pedestal, and the cultured cells can be punched out and collected together with the bottom member.

As described above, according to the culture vessel, it is possible to omit the time and effort of disassembling the members in a case of collecting the cells, as compared with a configuration in which the cell culture membrane is sandwiched and fixed between a plurality of members from above and below. In addition, since the number of members can be reduced, there is little risk of contamination.

In addition, the bottom member is formed of a degradable material. As a result, after collecting the cells together with the bottom member by a cell collection instrument, in a case where the bottom member is degraded, it is possible to use only the cells (for example, for transplantation into a living body).

Further, in the manufacturing method of the culture vessel, the bottom member is thermally fusion-welded to the main body portion. Therefore, it is possible to select a material according to the performance required for the bottom member. In addition, it is not necessary to use an adhesive or the like.

Furthermore, in the manufacturing method of a culture vessel, the bottom member is heated with a heat source that is disposed with a buffer member interposed between the bottom member and the heat source, and the bottom member is thermally fusion-welded to the lower end surface of the main body portion. In addition, the buffer member has non-adhesiveness such that the bottom member does not adhere to the buffer member at the melting temperature of the bottom member.

Therefore, it is possible to suppress adhesion of the melted bottom member to the heat source.

A manufacturing method of a culture vessel according to a twentieth aspect is the manufacturing method of a culture vessel according to the thirteenth aspect, in which the buffer member includes a fluororesin.

In the manufacturing method of a culture vessel according to the twentieth aspect, the buffer member includes a fluororesin. As a result, a desired effect can be obtained with a general-purpose material.

According to the present disclosure, it is possible to reduce the time and effort in collecting cells.

### BRIEF DESCRIPTION OF THE DRAWINGS

FIG. 1A is a perspective view showing a culture mechanism according to an embodiment of the present disclosure.
FIG. 1B is a vertical cross-sectional view showing a well plate and a culture vessel according to the embodiment of the present disclosure.
FIG. 1C is a cross-sectional view showing a state in which cells are cultured in the culture vessel according to the embodiment of the present disclosure.
FIG. 2A is a perspective view showing the culture vessel according to the embodiment of the present disclosure.
FIG. 2B is a perspective view showing a state in which an access instrument is inserted into the culture vessel according to the embodiment of the present disclosure.
FIG. 3A is a cross-sectional view showing a state in which the access instrument is inserted into the culture vessel according to the embodiment of the present disclosure.
FIG. 3B is a cross-sectional view taken along line B-B in FIG. 3A.
FIG. 3C is a cross-sectional view taken along line C-C in FIG. 3A.
FIG. 3D is a reference view showing a comparative example.
FIG. 4 is a perspective view showing an example of a manufacturing method of a culture vessel according to the embodiment of the present disclosure.
FIG. 5 is a cross-sectional view showing an arrangement example of a mold and the culture vessel in the manufacturing method of the culture vessel according to the embodiment of the present disclosure.
FIG. 6 is an enlarged vertical cross-sectional view and an enlarged horizontal cross-sectional view showing an arrangement example of the mold and the culture vessel in the manufacturing method of the culture vessel according to the embodiment of the present disclosure.
FIG. 7 is a perspective view showing an example of the culture vessel and a support member according to the embodiment of the present disclosure.
FIG. 8A is a cross-sectional view showing a state in which the culture vessel and the support member according to the embodiment of the present disclosure are combined.
FIG. 8B is a cross-sectional view showing a state in which the culture vessel and the support member according to the embodiment of the present disclosure are combined.
FIG. 9 is a perspective view showing another example of a culture vessel and a support member according to the embodiment of the present disclosure.
FIG. 10 is a cross-sectional view showing a state in which the culture vessel and the support member according to another example of the embodiment of the present disclosure are combined.
FIG. 11A is a cross-sectional view showing a modification of the culture vessel according to the embodiment of the present disclosure.
FIG. 11B is a cross-sectional view taken along line B-B in FIG. 11A.
FIG. 11C is a cross-sectional view taken along line C-C in FIG. 11A.
FIG. 11D is a cross-sectional view taken along line B-B in FIG. 11A in a case where an inner peripheral wall of the culture vessel according to the embodiment of the present disclosure is formed in a circular shape.
FIG. 11E is a cross-sectional view taken along line C-C in FIG. 11A in a case where the inner peripheral wall of the culture vessel according to the embodiment of the present disclosure is formed in a circular shape.

### DESCRIPTION OF THE PREFERRED EMBODIMENTS

Hereinafter, a culture mechanism, a culture vessel, and a manufacturing method of a culture vessel according to an embodiment of the present disclosure will be described with reference to the drawings. Components indicated by the same reference numerals in the drawings are the same components.

However, unless otherwise specified in the specification, each component is not limited to one, and a plurality of each component may be present.

In addition, description of overlapping configurations and reference numerals in the respective drawings may be omitted. The present disclosure is not limited to the embodiments described below, and may be implemented with appropriate modifications within the scope of the object of the present disclosure, such as omitting a configuration, replacing a configuration with a different configuration, or using one embodiment in combination with various modifications.

### <Culture Mechanism>

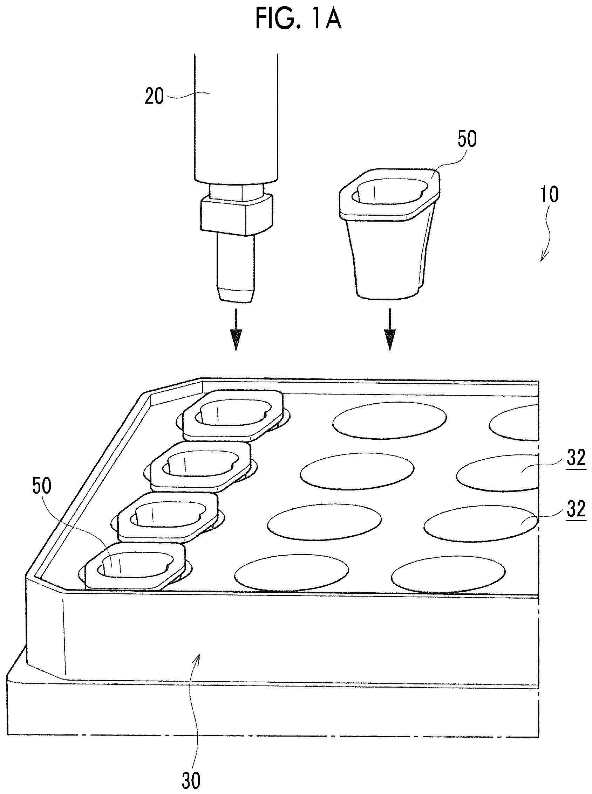

A culture mechanism 10 according to the embodiment of the present disclosure shown in FIG. 1A includes an access instrument 20, a well plate 30, and a culture vessel 50.

A plurality of recessed portions 32, which are bottomed holes, are formed in the well plate 30, and each of the recessed portions 32 is open to an upper surface of the well plate 30.

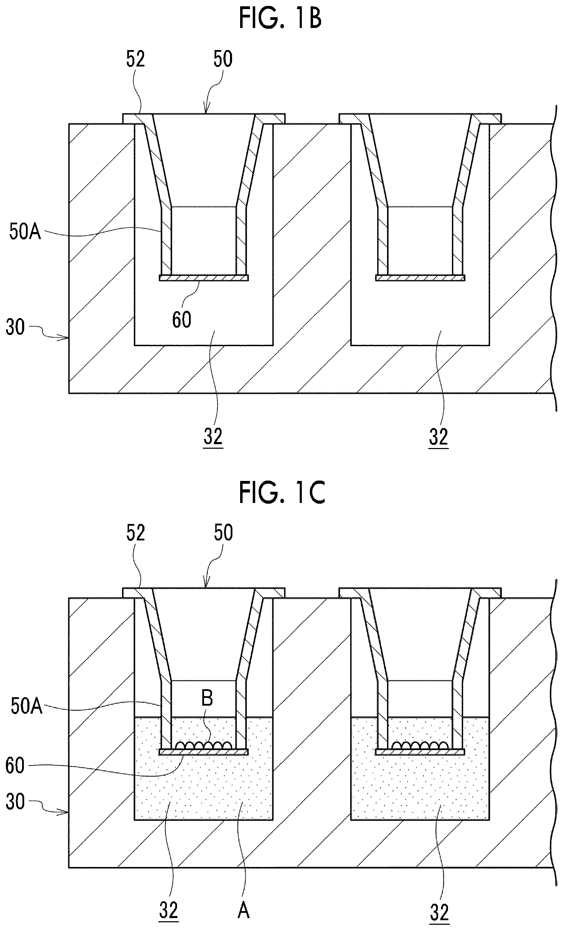

As shown in FIG. 1B, the culture vessel 50 is inserted into the recessed portion 32 from above. The culture vessel 50 includes a flange 52 that protrudes in a lateral direction from an opening end of an upper end portion. The flange 52 is placed on the opening end of the recessed portion 32. As a result, the culture vessel 50 is held in a state of being inserted into the recessed portion 32.

Although details will be described later, the culture vessel 50 includes a bottom member 60 at a lower end portion. The bottom member 60 is disposed to be spaced from a bottom surface of the recessed portion 32 in a state in which the culture vessel 50 is inserted into the recessed portion 32.

As shown in FIG. 1C, a culture solution A is accumulated in the recessed portion 32, and the bottom member 60 is immersed in the culture solution A, so that, for example, cells B can be cultured on the bottom member 60. That is, in the bottom member 60, a portion forming a bottom surface of the culture vessel 50 is a culture surface of the cells B.

### <Culture Vessel>

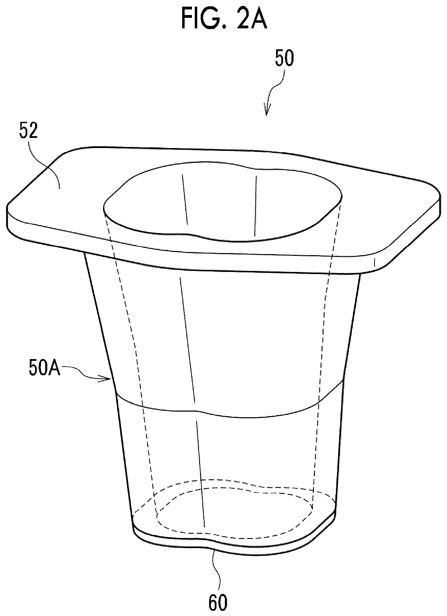

As shown in FIG. 2A, the culture vessel 50 has a main body portion 50A formed in a tubular shape. A material forming the main body portion 50A can be appropriately selected, but in the present embodiment, methyl methacrylate acrylonitrile butadiene styrene (MABS) is used from the viewpoint of thermal fusion-weldability, which will be described later.

The main body portion 50A has an opening at the upper end portion, and the flange 52 described above is formed at an opening end of the opening. In addition, the main body portion 50A also has an opening at a lower end portion, and the bottom member 60 is provided at an opening end of the opening.

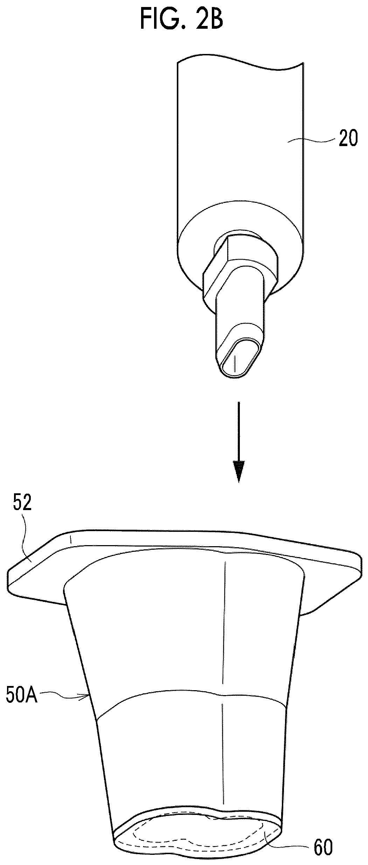

As shown in FIG. 2B, the access instrument 20 can be inserted into the main body portion 50A from the opening formed at an upper end portion of the main body portion 50A.

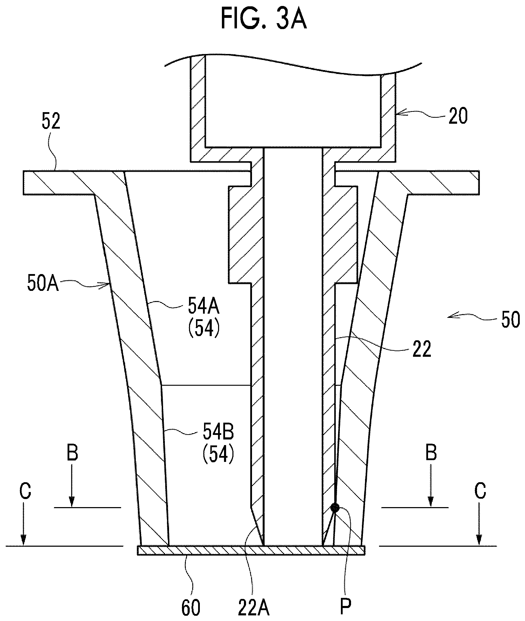

As shown in FIG. 3A, a cross-sectional area of a portion surrounded by an inner peripheral surface of a wall portion 54 forming the main body portion 50A in a tubular shape gradually decreases from the opening at the upper end portion of the main body portion 50A toward the bottom member 60. In other words, the wall portion 54 is formed to be inclined inward from the upper end portion to the lower end portion of the main body portion 50A. As a result, the main body portion 50A is formed in a mortar-like shape.

In addition, the wall portion 54 includes an upper wall portion 54A and a lower wall portion 54B. The upper wall portion 54A has a larger inclination angle with respect to a vertical direction than the lower wall portion 54B. Therefore, a distal end of the access instrument 20 is guided to a predetermined position on the bottom member 60 according to an insertion depth of the access instrument 20 into the main body portion 50A.

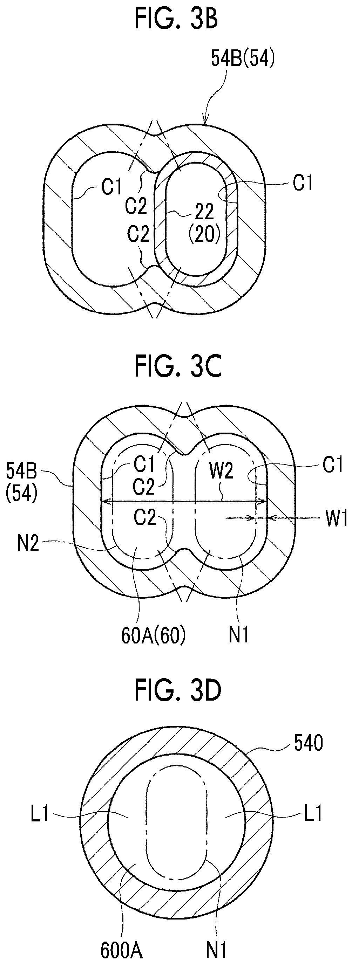

As shown in FIG. 3B, the wall portion 54 has a tubular shape formed by overlapping two identical ovals in a plan view. Specifically, the wall portion 54 has a tubular shape in which two ovals are disposed side by side in a longitudinal direction, sides facing each other in the lateral direction are omitted, and arc portions included in the ovals are connected to each other.

In other words, the inner peripheral surface of the wall portion 54 has two portions C1 along the ovals. In addition, the portions C1 are connected to each other by portions C2 that do not follow the ovals. In FIG. 3B, a boundary portion between the portion C1 and the portion C2 is indicated by a one-dot chain line.

As a result, the inner peripheral surface of the wall portion 54 has a shape in which an oval can be inscribed in each of the two portions C1.

### (Access Instrument)

The access instrument 20 is an instrument that is inserted into the main body portion 50A from the opening formed at the upper end portion of the main body portion 50A and is brought close to an upper surface of the bottom member 60. As the access instrument 20, various instruments such as a cell acquisition instrument, a pump, and a sensor can be adopted. In the present specification, a case where the access instrument 20 is used as the cell acquisition instrument will be mainly described.

In the access instrument 20, as shown in FIG. 3A, a tubular portion 22 having a uniform cross section along the longitudinal direction is formed at a distal end portion. In addition, a tapered portion 22A in which an outer peripheral surface is inclined toward an inner peripheral surface and a cross-sectional area gradually decreases is formed at a distal end of the tubular portion 22. By being pressed against the bottom member 60, the tapered portion 22A can punch out and collect the bottom member 60 and the cells B (see FIG. 1C) cultured on the bottom member 60.

An outer peripheral surface of the tubular portion 22 of the access instrument 20 is partially similar in shape to the inner peripheral surface of the wall portion 54 of the culture vessel 50. Specifically, the outer peripheral surface of the tubular portion 22 has an oval shape. On the other hand, the portion C1 along the oval on the inner peripheral surface of the wall portion 54 is similar to the oval forming the outer peripheral surface of the tubular portion 22.

At a point P shown in FIG. 3A, as shown in FIG. 3B, a part of the outer peripheral surface of the tubular portion 22 comes into contact with the portion C1 of the inner peripheral surface of the wall portion 54. In other words, at the point P, the outer peripheral surface of the tubular portion 22 in the access instrument 20 comes into contact with a part (that is, the portion C1) of the inner peripheral surface of the main body portion 50A.

As a result, as indicated by a one-dot chain line N1 in FIG. 3C, the distal end of the access instrument 20, that is, a distal end of the tapered portion 22A can be guided to a predetermined position (in other words, a position determined on the culture surface 60A) of the culture surface 60A in the bottom member 60. The point P is a base end portion of the tapered portion 22A.

The term "guide" means to guide the distal end of the tapered portion 22A to a predetermined position on the culture surface 60A by restricting the posture and position of the access instrument 20 in accordance with insertion of the access instrument 20 into the main body portion 50A.

For example, in the present embodiment, the posture of the access instrument 20 is restricted such that the distal end of the tapered portion 22A in the access instrument 20 is parallel to the culture surface 60A in accordance with the insertion of the access instrument 20 into the main body portion 50A. In addition, a part of the outer peripheral surface of the tubular portion 22 in the access instrument 20 comes into contact with the portion C1 of the inner peripheral surface of the wall portion 54, and the position is restricted. Then, the distal end of the tapered portion 22A is guided to a predetermined position indicated by the one-dot chain line N1 in FIG. 3C on the culture surface 60A.

That is, the posture of the access instrument 20 and the distal end of the tapered portion 22A are uniquely positioned regardless of the technical skill of an operator.

Here, the "culture surface 60A" refers to an inner portion of the wall portion 54 in the bottom member 60. The culture surface 60A has a non-circular shape formed by combining a portion along an oval and a portion not along the oval. In other words, a portion surrounded by the inner peripheral surface of the wall portion 54 has a non-circular shape.

The culture surface 60A is, at least in part, offset (positional shift) outward at equal intervals from the distal end of the access instrument 20 (portion indicated by the one-dot chain line N1). In other words, the distal end of the access instrument 20 is disposed along a portion offset inward at equal intervals from the portion C1 of the inner peripheral surface of the wall portion 54.

In addition, in a state in which the outer peripheral surface of the tubular portion 22 is in contact with the inner peripheral surface of the wall portion 54 at the point P shown in FIG. 3A, a minimum distance W1 between an edge of the culture surface 60A shown in FIG. 3C and the distal end of the access instrument 20 is more than 0.2% and less than 20% with respect to a maximum dimension W2 between the edges of the culture surface 60A.

As a specific example, the minimum distance W1 is about 0.1 mm, the maximum dimension W2 is about 2 mm, and the minimum distance W1 is about 5% with respect to the maximum dimension W2.

In addition, contact portions with which the outer peripheral surface of the tubular portion 22 is configured to come into contact are formed at a plurality of locations on the inner peripheral surface of the wall portion 54. Specifically, two portions C1 shown in FIG. 3B are contact portions with which the outer peripheral surface of the tubular portion 22 can be in contact.

Each of the portions C1 guides the distal end of the access instrument 20 to a different position on the culture surface 60A. Specifically, the portion C1 on the right side of the paper of FIG. 3B guides the distal end of the access instrument 20 to a portion indicated by the one-dot chain line N1 in FIG. 3C. On the other hand, the portion C1 on the left side of the paper of FIG. 3B guides the distal end of the access instrument 20 to a portion indicated by a one-dot chain line N2 in FIG. 3C.

### (Bottom Member)

The bottom member 60 is formed of a degradable material. The "degradable material" refers, for example, to a material that is hydrolyzed. Examples of the material that is hydrolyzed include a material that is hydrolyzed in a living body (hereinafter, referred to as "biodegradation"). In addition, preferably, the bottom member 60 is formed of a biocompatible material. The "biocompatible material" is a material that is unlikely to cause side effects or the like in a living body, regardless of whether the material is biodegradable.

In the present aspect, an example in which polylactide-co-glycolide (PLGA) that is a biodegradable material is used as the bottom member 60 will be described. As the PLGA, PLGA having a DL-lactide/glycolide ratio of about 1:1 can be used. In addition, from the viewpoint of fusion weldability, the surface free energy of MABS forming the main body portion 50A is set to -10 mJ/m² or more and +10 mJ/m² or less with respect to the surface free energy of PLGA. From the viewpoint of improving the fusion weldability, the surface of MABS may be subjected to a plasma treatment or may be provided with fine irregularities.

Further, the bottom member 60 is formed as a porous body having a thickness of 20 to 50 µm by overlapping and disposing membrane bodies woven from fibrous PLGA (so-called nanofibers) having a diameter of about 150 to about 650 nm such that an average opening is 1 µm.

As shown in FIG. 3A, the bottom member 60 is adhered to a lower end surface of the wall portion 54 in the main body portion 50A. A method of adhering the bottom member 60 to the main body portion 50A is not particularly limited, but thermal fusion-welding is used in the present embodiment. A method of thermal fusion-welding is also not particularly limited, and examples thereof include a method using a heat block and a method using ultrasound waves or lasers. However, in the present embodiment, a heat block is used.

### (Thermal Fusion-Welding Method)

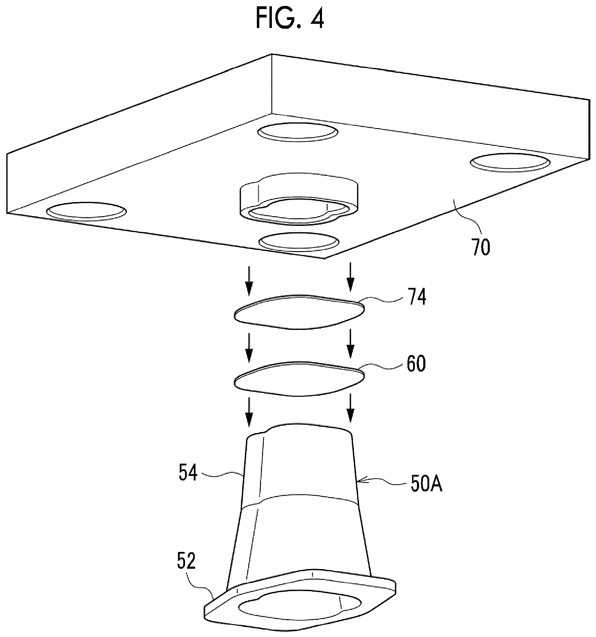

In order to thermally fusion-weld the bottom member 60 to the main body portion 50A, for example, as shown in FIG. 4, a mold 70 as a heat block and a fluororesin sheet 74 as a buffer member are used.

Specifically, first, the wall portion 54 in the main body portion 50A is disposed by inverting the upper and lower parts with respect to the use posture (in other words, the wall portion 54 is disposed such that the lower end surface of the wall portion 54 in the posture during cell culture is the upper end surface).

Then, the bottom member 60 is disposed on an upper end surface of the wall portion 54 in this posture. As the bottom member 60, a bottom member having a size that protrudes outward from the outer edge of the upper end surface of the wall portion 54 is used. In addition, the bottom member 60 is disposed to cover an opening of the main body portion 50A surrounded by the wall portion 54.

Then, the mold 70 is heated in a state in which the fluororesin sheet 74 is sandwiched between the bottom member 60 and the mold 70, and heat is transferred from the mold 70 to the bottom member 60. Then, the bottom member 60 is pressed against the wall portion 54. As a result, the bottom member 60 is thermally fusion-welded to the main body portion 50A. In the bottom member 60, a portion that protrudes outward from the main body portion 50A may be appropriately cut off.

In a case of the thermal fusion-welding, the temperature of the mold 70 is set to 200°C, the pressing force is set to 20 N, and the pressing time is set to 1 second. These values temperature, pressing force, and pressing time can be appropriately changed according to one another. In addition, the thickness of the fluororesin sheet 74 is not particularly limited, but is, for example, 75 µm. This thickness can be appropriately changed depending on heating conditions or pressing conditions.

It is preferable that the fluororesin sheet 74 covers the entire surface of the bottom member 60. As a result, it is possible to suppress the transmission of radiant heat from the mold 70 to the bottom member 60. As the buffer member, a material other than the fluororesin may be used. However, it is suitable to use a material having non-adhesiveness such that the bottom member 60 does not adhere to the member at the melting temperature of the bottom member 60.

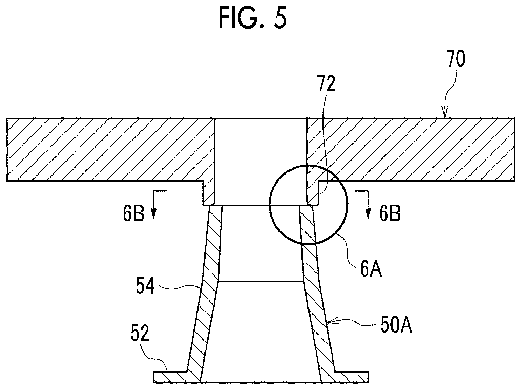

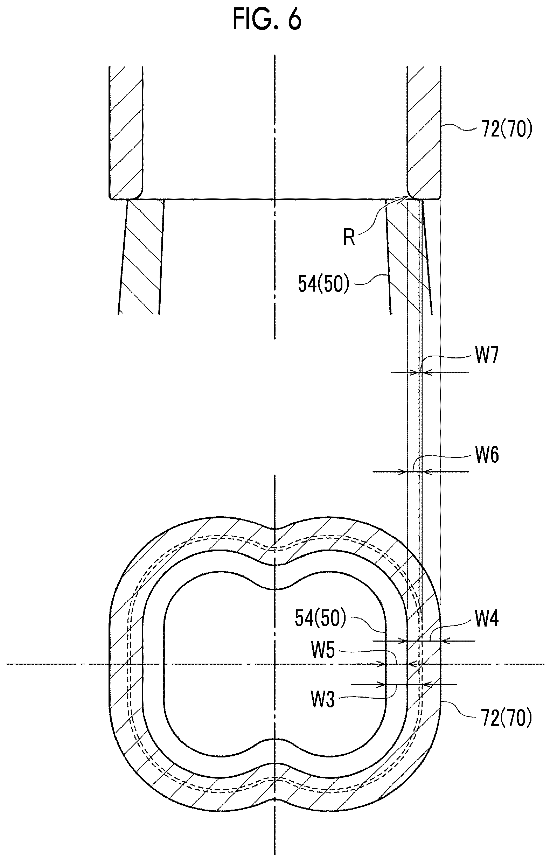

Here, FIGS. 5 and 6 show a positional relationship between the mold 70 and the wall portion 54 of the main body portion 50A in a case where the main body portion 50A is pressed by the mold 70. In these drawings, illustration of the bottom member 60 and the fluororesin sheet 74 is omitted. FIG. 6 shows an enlarged view of a region indicated by 6A in FIG. 5 and a cross-sectional view of a portion indicated by a line 6B-6B in FIG. 5.

As shown in FIG. 5, in the mold 70, a heating portion 72 facing the wall portion 54 does not face an inner portion of an end surface of the wall portion 54, but faces an outer portion thereof.

Specifically, a thickness W3 of the wall portion 54 shown in FIG. 6 is, for example, 1.0 mm, and a thickness W4 of the heating portion 72 is, for example, 0.9 mm. The heating portion 72 is disposed to be offset outward with respect to the wall portion 54 by a width W5. The width W5 is, for example, 0.6 mm. As a result, an overlapping width W6 between the wall portion 54 and the heating portion 72 is 0.4 mm.

In addition, a chamfer R is formed between a lower end surface of the heating portion 72 and an inner peripheral wall, and a radius thereof is 0.3 mm. As a result, in the overlapping width W6 between the wall portion 54 and the heating portion 72, a thickness W7 of a portion in which the wall portion 54 and the lower end surface of the heating portion 72 face each other in parallel is 0.1 mm.

### (Support Member)

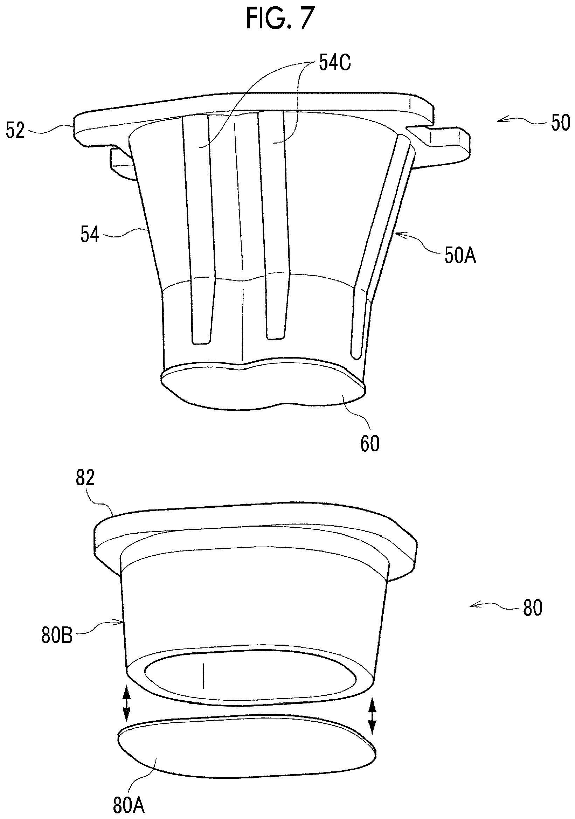

A support member 80 shown in FIG. 7 can be mounted on the culture vessel 50. The support member 80 comprises a support portion 80A and a holding portion 80B.

The support portion 80A is formed of a porous body having a pore diameter of about 0.4 µm, and is a membrane material having higher rigidity than the bottom member 60. A material forming the support portion 80A is not particularly limited, and for example, polycarbonate or the like can be used. The support portion 80A is thermally fusion-welded to a lower end surface of the holding portion 80B.

The phrase "higher rigidity than the bottom member 60" means that the support portion 80A is less likely to deflect in an out-of-plane direction (downward direction in a culture posture of cells) than the bottom member 60.

The holding portion 80B is a tubular member that holds the support portion 80A below the bottom member 60. The holding portion 80B has an opening at an upper end portion, and a flange 82 that protrudes in a lateral direction is provided at an opening end of the upper end portion. In addition, the holding portion 80B also has an opening at a lower end portion, and the support portion 80A is provided at this opening end.

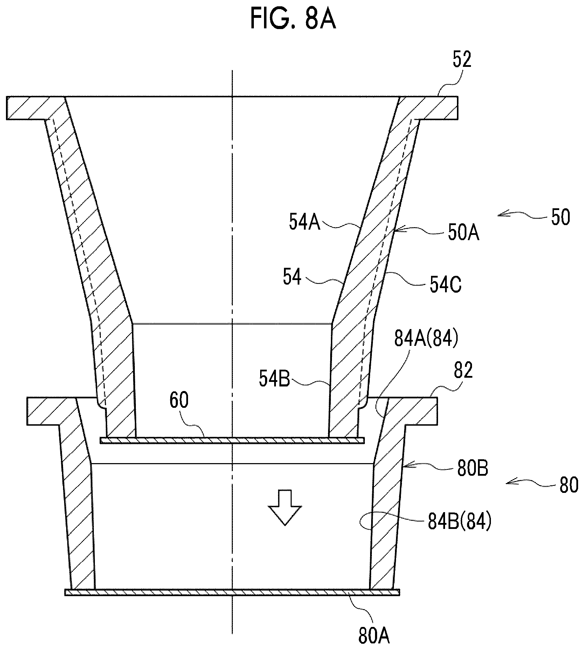

As shown in FIG. 8A, the culture vessel 50 can be inserted into the opening formed at the upper end portion of the holding portion 80B. A cross-sectional area of a portion surrounded by an inner peripheral surface of a wall portion 84 forming the holding portion 80B in a tubular shape gradually decreases from the opening at the upper end portion of the holding portion 80B toward the support portion 80A. In other words, the wall portion 84 is formed to be inclined inward from the upper end portion to the lower end portion of the holding portion 80B. As a result, the holding portion 80B is formed in a mortar-like shape.

In addition, the wall portion 84 includes an upper wall portion 84A and a lower wall portion 84B. The upper wall portion 84A has a larger inclination angle with respect to the vertical direction than the lower wall portion 84B.

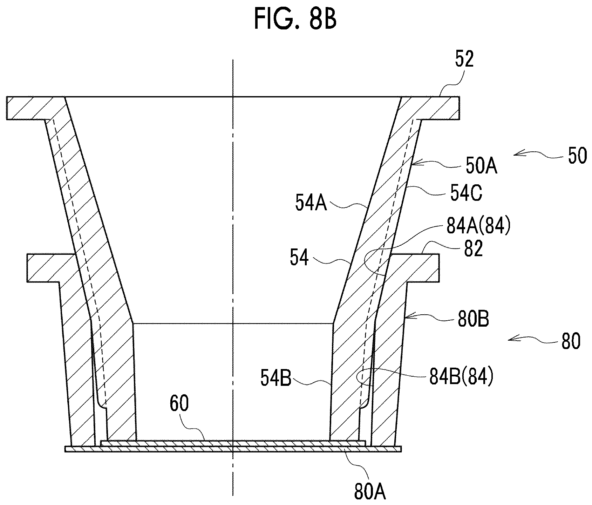

As shown in FIG. 8B, in a case where the culture vessel 50 is inserted into the holding portion 80B of the support member 80, the upper wall portion 54A of the main body portion 50A in the culture vessel 50 is disposed along and in contact with the upper wall portion 84A of the holding portion 80B in the support member 80.

In addition, in this state, the bottom member 60 of the culture vessel 50 is disposed close to or in contact with the support portion 80A of the support member 80. In this way, the holding portion 80B is fitted to the main body portion 50A to hold the support portion 80A below the bottom member 60.

It should be noted that, regardless of whether the support member 80 is used, the wall portion 54 of the culture vessel 50 may be provided with a rib 54C along the up-down direction. FIG. 2A shows an example in which the rib 54C is not provided, and FIG. 7 shows an example in which the rib 54C is provided.

Since torsional rigidity of the wall portion 54 is increased by providing the rib 54C, it is possible to suppress deformation of the bottom member 60 bonded to the wall portion 54. In addition, by providing the rib 54C, the rib 54C comes into contact with the upper wall portion 84A of the holding portion 80B. As a result, as compared with a case where the rib 54C is not provided, a contact area between the culture vessel 50 and the support member 80 is reduced, and a compressive stress acts locally between the culture vessel 50 and the support member 80. As a result, the culture vessel 50 and the support member 80 are fixed to each other such that the rib 54C bites into the support member 80.

### (Modification of Support Member)

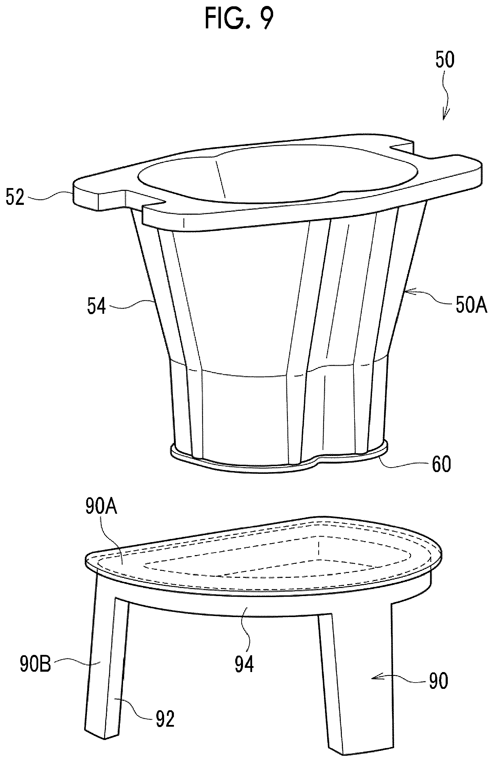

The culture vessel 50 can also be supported by a support member 90 shown in FIG. 9. The support member 90 comprises a support portion 90A and a holding portion 90B.

The support portion 90A is formed of a porous body having a pore diameter of about 0.4 µm, and is a membrane material having higher rigidity than the bottom member 60. A material forming the support portion 90A is not particularly limited, and for example, polycarbonate or the like can be used. The support portion 90A is thermally fusion-welded to an upper end surface of the holding portion 90B.

The holding portion 90B is a pedestal that holds the support portion 90A below the bottom member 60. The holding portion 90B has three legs 92 and an annular member 94 supported by the legs 92. The support portion 90A is thermally fusion-welded to an upper end surface of the annular member 94 and is supported from below by the holding portion 90B.

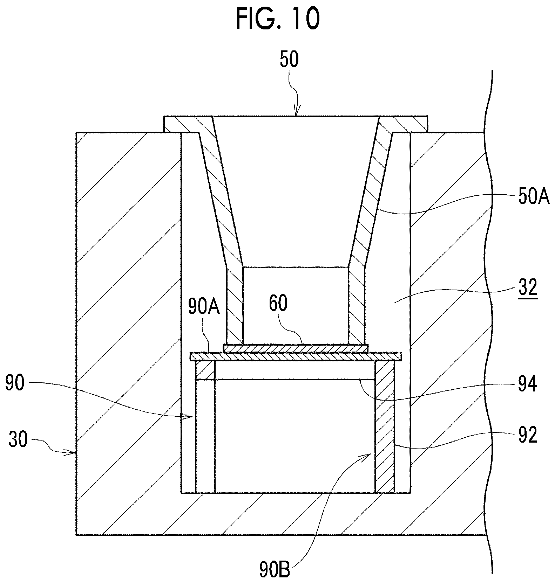

In a case where the support member 90 is used, as shown in FIG. 10, the support member 90 is disposed in the recessed portion 32 of the well plate 30, and the culture vessel 50 is disposed above the support member 90. For the support member 90, it is preferable to use a material having a higher specific gravity than the culture solution A (see FIG. 1C). The holding portion 90B may have a configuration in which the annular member 94 is omitted and the support portion 90A is held only by the legs 92.

### <Action and Effect>

In the culture vessel 50 according to the embodiment of the present disclosure, as shown in FIG. 1C, the cells B can be cultured on the bottom member 60 at the lower end portion of the main body portion 50A. Then, as shown in FIG. 3A, the access instrument 20 for accessing the culture surface can be brought close to the cultured cells B (illustration of the cells B is omitted in the drawings other than FIG. 1C).

For example, in a case where a cell collection instrument is used as the access instrument 20, the cells B can be collected with the cell collection instrument that is inserted from the upper end portion of the tubular main body portion 50A. In this case, the outer peripheral surface of the tubular portion 22 in the access instrument 20 (the outer peripheral surface indicated by the point P in FIG. 3A) comes into contact with at least a part of the inner peripheral surface of the culture vessel 50 (the portion C1 in FIG. 3B). Then, the distal end of the access instrument 20 indicated by the one-dot chain line N1 in FIG. 3C is guided to a predetermined position on the culture surface 60A.

That is, the access range of the access instrument 20 to the culture surface 60A is positioned. In a case where a cell acquisition instrument is used as the access instrument 20, a cell collection range is positioned. As a result, the cell collection operation can be easily performed, and the operator dependency and the error of the operation are reduced. In addition, the cell collection range for the culture surface 60A is relatively increased. As described above, the yield of cells can be increased.

On the other hand, in a case where the distal end of the access instrument is not guided to a predetermined position on the culture surface and the access range is not positioned, for example, the cell collection position is not determined, so that a deviation may occur during cell collection, which may affect the integrity of cell tissue. Therefore, the cell collection operation is difficult. In addition, by the amount that a movable range of the distal end of the cell collection instrument is secured on the culture surface, the cell collection range to the culture surface is relatively narrowed, and the yield of cells is reduced.

In addition, in the culture vessel 50 according to the embodiment of the present disclosure, as shown in FIG. 3C, the culture surface 60A is, at least in part, offset outward at equal intervals from the distal end of the access instrument 20. In other words, at least a part of the inner peripheral surface of the wall portion 54 of the culture vessel 50 is disposed at a position offset outward at equal intervals from the distal end of the access instrument 20.

For example, in the present embodiment, the distal end of the access instrument 20 has an oval shape, and the culture surface 60A has a portion offset outward at equal intervals along the oval shape. As a result, the yield of cells can be increased.

On the other hand, as shown in "Comparative Example" of FIG. 3D, in a case where the culture surface 600A is not offset outward at equal intervals from the distal end N1 of the access instrument 20, for example, in a case where the distal end of the access instrument 20 has an oval shape and the culture surface 600A (inner peripheral surface of the wall portion 540) has a circular shape, a region where cells cannot be collected (for example, a region L1) is increased in a short-side direction of the oval. Therefore, it is difficult to increase the yield of cells.

In addition, in the culture vessel 50 according to the embodiment of the present disclosure, the minimum distance W1 between the edge of the culture surface 60A and the distal end of the access instrument is more than 0.2% with respect to the maximum dimension W2 between the edges. Therefore, as compared with a case of 0.2% or less, a clearance around the distal end of the cell collection instrument is widened, and thus manufacturing tolerances of the culture vessel 50 and the access instrument 20 are easily absorbed.

In addition, according to the culture vessel 50, the minimum distance W1 between the edge of the culture surface and the distal end of the access instrument is less than 20% with respect to the maximum dimension W2 between the edges. Therefore, as compared with a case of 20% or more, the clearance around the distal end of the access instrument 20 is narrowed, and thus the yield of cells can be increased.

In addition, in the culture vessel 50 according to the embodiment of the present disclosure, contact portions (portions C1) with which the outer peripheral surface of the tubular portion 22 is configured to come into contact are formed at a plurality of locations on the inner peripheral surface of the wall portion 54. Each of the portions C1 guides the distal end of the access instrument 20 to different positions on the culture surface indicated by one-dot chain lines N1 and N2 in FIG. 3C. As a result, a plurality of cell pieces can be collected from one culture surface 60A.

In addition, in the culture vessel 50 according to the embodiment of the present disclosure, the culture surface 60A has a non-circular shape formed by combining portions along an oval. As a result, it is easy to collect a cell piece having an oval shape.

In addition, in the culture vessel 50 according to the embodiment of the present disclosure, as shown in FIG. 3A, the cross-sectional area of the portion surrounded by the inner peripheral surface of the wall portion 54 forming the main body portion 50A in a tubular shape gradually decreases from the opening at the upper end portion of the main body portion 50A toward the bottom member 60. As a result, it is easy to guide the access instrument 20 that is inserted from the opening at the upper end portion of the main body portion 50A to the culture surface 60A.

In addition, in the culture vessel 50 according to the embodiment of the present disclosure, the bottom member 60 is formed of a degradable material. As a result, after collecting the cells B (see FIG. 1C) together with the bottom member 60 by the access instrument, in a case where the bottom member 60 is degraded, it is possible to use only the cells (for example, transplantation into a living body).

In addition, in the culture vessel 50 according to the embodiment of the present disclosure, the bottom member 60 is degraded by biodegradation. As a result, after collecting the cells B together with the bottom member 60 by the access instrument 20, even in a case where the cells are transplanted into a living body together with the bottom member 60, the bottom member 60 is degraded in the living body, and only the cells are transplanted. That is, the bottom member 60, which is undegraded during collection and transplantation of the cells B, is degraded in a living body after transplantation into the living body.

In addition, in the culture vessel 50 according to the embodiment of the present disclosure, the bottom member 60 is formed as a porous body by overlapping and disposing membrane bodies woven from fibrous PLGA.

Therefore, as shown in FIG. 1C, the culture vessel 50 is immersed in the culture solution A, so that the bottom member 60 is infused with the culture solution A, and the bottom member 60 is immersed in the culture solution A. Therefore, the culture solution A is supplied to the bottom member 60 from the up-down direction. As a result, the culture efficiency of the cells B is improved. On the other hand, in a case where the bottom member is not porous, the culture solution A is supplied only from one side of the bottom member, so that the culture efficiency is relatively poor.

In addition, in the culture vessel 50 according to the embodiment of the present disclosure, as shown in FIG. 1B, the bottom member 60 is fixed to the lower end portion of the main body portion 50A and constitutes the bottom of the main body portion 50A. Therefore, in a case where the culture vessel 50 is placed on a pedestal or the like, a cell collection instrument as an example of the access instrument 20 is inserted from the upper end portion of the tubular main body portion 50A, and a distal end of the cell collection instrument is pressed against the bottom member 60, a reaction force is obtained from the pedestal, and the cultured cells B (see FIG. 1C) can be punched out and collected together with the bottom member 60.

As described above, according to the culture vessel 50, it is possible to omit the time and effort required to disassemble the members in a case of collecting the cells B, as compared with a configuration in which a cell culture membrane is fixed by being sandwiched from above and below by a plurality of members. That is, it is possible to reduce the time and effort required to collect the cells B. In addition, since the number of members can be reduced, there is little risk of contamination.

In addition, in the culture vessel 50 according to the embodiment of the present disclosure, the bottom member 60 is adhered to the lower end surface of the main body portion 50A. That is, the bottom member 60 is formed as a separate member from the main body portion 50A. Therefore, it is possible to select a material (in the present embodiment, PLGA) according to a performance required as the bottom member 60.

In addition, in the culture vessel 50 according to the embodiment of the present disclosure, the bottom member 60 is thermally fusion-welded to the main body portion 50A. Therefore, it is not necessary to use an adhesive or the like. As a result, since no other material is interposed between the bottom member 60 and the main body portion 50A, the number of components can be reduced.

In addition, in the culture vessel 50 according to the embodiment of the present disclosure, the bottom member 60 is disposed in the positional relationship shown with respect to the mold 70 shown in FIG. 6 during thermal fusion-welding. As a result, the bottom member 60 is thermally fusion-welded to an outer portion of the lower end surface of the main body portion 50A from a position offset outward from the culture surface 60A. Therefore, it is possible to reduce the influence of heat on the culture surface 60A as compared with a case where no offset is provided.

In addition, in the culture vessel 50 according to the embodiment of the present disclosure, a surface free energy of the main body portion 50A is -10 mJ/m² or more and +10 mJ/m² or less with respect to a surface free energy of the bottom member 60. As a result, the bondability by thermal fusion-welding is improved as compared with a case where the surface free energy of the main body portion 50A is smaller than -10 mJ/m² or larger than +10 mJ/m² with respect to the surface free energy of the bottom member 60.

In addition, the culture vessel 50 according to the embodiment of the present disclosure can be used in combination with the support member 80 shown in FIG. 7 and the support member 90 shown in FIG. 9. As a result, the deflection of the bottom member 60 can be reduced.

Among these, in the support member 80, the holding portion 80B is fitted to the main body portion 50A of the culture vessel 50. Therefore, the support portion 80A can be held below the bottom member 60 without relying on other members.

On the other hand, in the support member 90, the holding portion 90B is a pedestal that supports the support portion 90A from below. Therefore, the support portion 90A can be held below the bottom member 60 with a simple configuration as compared with a case where the holding portion of the support member is fitted to the main body portion.

In addition, the support portion 80A of the support member 80 and the support portion 90A of the support member 90 are formed of a porous body. Therefore, as shown in FIG. 1C, in a case where the culture solution A is supplied from the lower side of the bottom member 60, the support portions 80A and 90A are infused with the culture solution A. Therefore, the supply of the culture solution to the bottom member 60 is less likely to be hindered by the support portions 80A and 90A.

In addition, in the manufacturing method of the culture vessel 50 according to the embodiment of the present disclosure (the method of thermally fusion-welding the bottom member 60 to the main body portion 50A), as shown in FIG. 4, the bottom member 60 is heated by a heat source (mold 70) disposed with a fluororesin sheet 74 as a buffer member sandwiched between the heat source and the bottom member 60, and the bottom member 60 is thermally fusion-welded to the lower end surface of the main body portion 50A.

In addition, the fluororesin sheet 74 has non-adhesiveness such that the bottom member 60 does not adhere at the melting temperature of the bottom member 60. Therefore, it is possible to suppress adhesion of the melted bottom member 60 to the mold 70.

In addition, in the manufacturing method of the culture vessel 50 according to the embodiment of the present disclosure, the fluororesin sheet 74 is used as the buffer member. As a result, an effect of suppressing adhesion of the melted bottom member 60 to the mold 70 by using a general-purpose material can be obtained.

### <Other Embodiments>

In the above-described embodiment, the culture surface 60A of the culture vessel 50 is, at least in part, offset outward at equal intervals from the distal end of the access instrument 20, but the embodiment of the present disclosure is not limited thereto.

For example, even in a case where the outer peripheral surface of the access instrument 20 comes into contact with the main body portion 50A of the culture vessel 50 and the distal end of the access instrument 20 is guided to a predetermined position on the culture surface 60A, it is not always necessary that the culture surface 60A be offset at "equal intervals". That is, the shape of the distal end of the access instrument 20 and the shape of the culture surface 60A can be appropriately selected according to the required shape of the cell piece.

In addition, in the above-described embodiment, a part of the outer peripheral surface of the tubular portion 22 in the access instrument 20 comes into contact with the portion C1 of the inner peripheral surface of the wall portion 54, and the posture of the access instrument 20 and the distal end of the tapered portion 22A are "uniquely" positioned, but the embodiment of the present disclosure is not limited thereto.

For example, in a state in which a part of the outer peripheral surface of the tubular portion 22 is in contact with the portion C1 of the inner peripheral surface of the wall portion 54, the distal end of the tapered portion 22A may be guided to a "shifted position" shifted from the position indicated by the one-dot chain line N1 in FIG. 3C on the culture surface 60A.

That is, in a state in which the distal end of the tapered portion 22A is in contact with the culture surface 60A, a movable range (play) may be secured between the access instrument 20 and the main body portion 50A. Even in such an aspect, the movable range of the access instrument 20 is restricted to the movable amount within the offset range described above. Therefore, even in a case where a hand shake or the like occurs, it is easy to collect a cell piece having a desired size.

In addition, as described above, a method of guiding the distal end of the access instrument 20 to a predetermined position on the culture surface 60A is to bring a part of the outer peripheral surface of the tubular portion 22 in the access instrument 20 into contact with the portion C1 of the inner peripheral surface of the wall portion 54.

In the present aspect, the outer peripheral surface of the tubular portion 22 in the access instrument 20 (the outer peripheral surface indicated by the point P in FIG. 3A) is in "line contact" with the portion C1 of the culture vessel 50, but the embodiment of the present disclosure is not limited thereto.

For example, a method of fixing any three points in the access instrument 20 to the inner peripheral portion of the main body portion 50A (point contact), a method of fixing any surface in the access instrument 20 to the inner peripheral surface of the main body portion 50A (surface contact), or the like may be adopted.

In addition, in the above-described embodiment, contact portions with which the outer peripheral surface of the access instrument 20 is configured to come into contact are formed at "a plurality of locations" on the inner peripheral surface of the main body portion 50A in the culture vessel 50, but the embodiment of the present disclosure is not limited thereto.

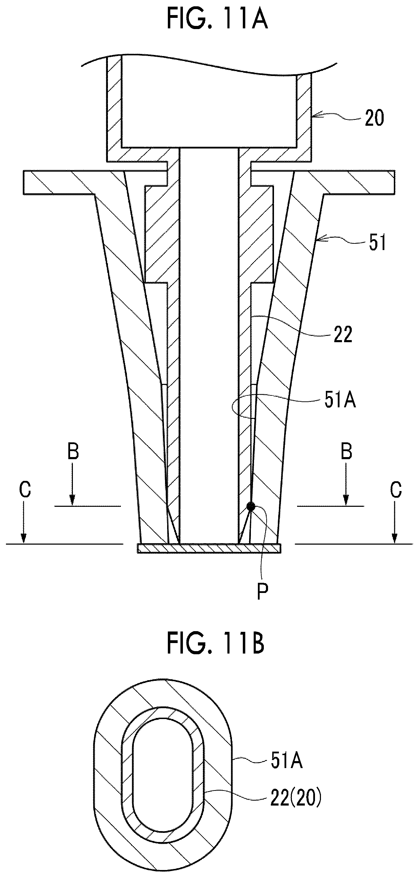

For example, as in a culture vessel 51 shown in FIGS. 11A to 11C, a contact portion with which the outer peripheral surface of the access instrument 20 is configured to come into contact may be formed at "only one location" on an inner peripheral surface of a wall portion 51A.

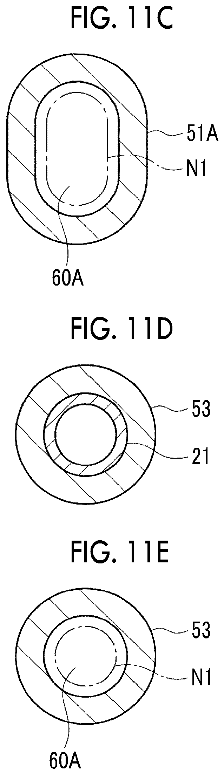

In such an aspect, with respect to the outer peripheral surface of the access instrument 20 having an oval shape, the inner peripheral surface of the wall portion 51A of the culture vessel 51 also has an oval inner peripheral surface. At a point P shown in FIG. 11A, as shown in FIG. 11B, the inner peripheral surface of the wall portion 51A comes into contact with the outer peripheral surface of the access instrument 20. In addition, as shown in FIG. 11C, the entire culture surface 60A is offset outward at equal intervals from the distal end of the access instrument 20.

In addition, in the above-described embodiment, the inner peripheral surface (culture surface 60A) of the wall portion 54 in the culture vessel 50 and the outer peripheral surface of the tubular portion 22 of the access instrument 20 are non-circular, but the embodiment of the present disclosure is not limited thereto. For example, as in an inner peripheral surface of a culture vessel 53 and an outer peripheral surface of an access instrument 21 shown in FIGS. 11D and 11E, these may be circular in shape.

In addition, in the above-described embodiment, the cross-sectional area of the portion surrounded by the inner peripheral surface of the wall portion 54 in the culture vessel 50 gradually decreases from the opening at the upper end portion of the main body portion 50A toward the culture surface 60A, but the embodiment of the present disclosure is not limited thereto. For example, the wall portion 54 may have a tubular shape in which the cross-sectional area of the portion surrounded by the inner peripheral surface is constant.

Further, in the above-described embodiment, the outer peripheral surface of the access instrument 20 comes into contact with at least a part of the inner peripheral surface of the main body portion 50A, and the distal end of the access instrument 20 is guided to a predetermined position on the culture surface 60A. However, the embodiment of the present disclosure is not limited thereto, and the outer peripheral surface of the access instrument 20 does not need to come into contact with the inner peripheral surface of the main body portion 50A.

In addition, in the above-described embodiment, the bottom member 60 is a porous body, but the embodiment of the present disclosure is not limited thereto. For example, in a case where the culture solution A shown in FIG. 1C is injected into the culture vessel 50 from above the bottom member 60, or in a case where the entire culture vessel 50 is immersed in the culture solution A, the cells B can be cultured even in a case where the bottom member 60 is not formed as a porous body.

That is, the bottom member 60 does not need to be formed by overlapping and disposing membrane bodies woven from fibrous PLGA, and may be formed of a membrane material having no mesh opening, or does not need to be formed of PLGA, and it is possible to select an appropriate material according to a purpose.

In addition, in the above-described embodiment, the bottom member 60 is thermally fusion-welded to an outer portion of the lower end surface of the main body portion 50A from a position offset outward from the culture surface 60A, but the embodiment of the present disclosure is not limited thereto. For example, the bottom member 60 may be thermally fusion-welded to the entire lower end surface of the main body portion 50A. The same applies to a case where an adhesion method other than thermal fusion-welding is used.

In addition, in the above-described embodiment, the bottom member 60 is adhered to the lower end surface of the main body portion 50A, but the embodiment of the present disclosure is not limited thereto. For example, in a case where the material of the bottom member 60 is a rigid member (for example, polycarbonate) equivalent to the main body portion 50A, the bottom member 60 may be fitted and fixed to the main body portion 50A.

In addition, in the above-described embodiment, the surface free energy of the main body portion 50A is set to -10 mJ/m² or more and +10 mJ/m² or less with respect to the surface free energy of the bottom member 60, but the embodiment of the present disclosure is not limited thereto. In a case where the bottom member 60 is not thermally fusion-welded to the lower end surface of the main body portion 50A, the surface free energy can be freely set.

In addition, in the above-described embodiment, the bottom member 60 is formed of a degradable material, but the embodiment of the present disclosure is not limited thereto. Even in a case where the bottom member 60 is not formed of a degradable material, the cells B can be cultured on the surface of the bottom member 60.

In addition, in the above-described embodiment, the configuration in which the culture vessel 50 includes the bottom member 60 has been described, but the culture vessel of the present disclosure may be a culture vessel in which an opening that is coverable by the bottom member 60 is formed at the lower end portion of the main body portion 50A, and the culture vessel does not necessarily include the bottom member 60. As described above, the present disclosure can be implemented in various aspects.

### <Supplementary Note 1>

(((1))) A culture vessel comprising:
   a main body portion that is formed in a tubular shape and that has an opening at an upper end portion; and
   a bottom member that is fixed to a lower end portion of the main body portion to constitute a bottom of the main body portion, and that is formed with a culture surface on which cells are culturable,
   in which the bottom member is formed of a degradable material.
(((2))) The culture vessel according to (((1))),
   in which the bottom member is adhered to a lower end surface of the main body portion.
(((3))) The culture vessel according to (((2))),
   in which the bottom member is thermally fusion-welded to the lower end surface.
(((4))) The culture vessel according to (((3))),
   in which the bottom member is thermally fusion-welded to an outer portion of the lower end surface from a position offset outward from the culture surface.
(((5))) The culture vessel according to (((3))),
   in which a surface free energy of the main body portion is -10 mJ/m² or more and +10 mJ/m² or less with respect to a surface free energy of the bottom member.
(((6))) The culture vessel according to any one of (((1))) to (((5))), further comprising:
   a support member that includes
   a support portion having higher rigidity than the bottom member, and
   a holding portion that holds the support portion below the bottom member.
(((7))) The culture vessel according to (((6))),
   in which the holding portion is fitted to the main body portion to hold the support portion.
(((8))) The culture vessel according to (((6))),
   in which the holding portion is a pedestal that supports the support portion from below.
(((9))) The culture vessel according to any one of (((1))) to (((8))),
   in which the bottom member is a porous body.
(((10))) The culture vessel according to any one of (((1))) to (((9))),
   in which the bottom member is formed of a fibrous material.
(((11))) The culture vessel according to (((9))) or (((10))), further comprising:
   a support member that includes
   a support portion having higher rigidity than the bottom member, and
   a holding portion that holds the support portion below the bottom member,
   in which the support portion is formed of a porous body.
(((12))) The culture vessel according to any one of (((1))) to (((11))),
   in which the bottom member is a material that is degraded by biodegradation.
(((13))) The culture vessel according to (((1))),
   in which an outer peripheral surface of an access instrument for accessing the culture surface, the access instrument being inserted from the opening at the upper end portion, comes into contact with at least a part of an inner peripheral surface of the main body portion so as to guide a distal end of the access instrument to a predetermined position on the culture surface.
(((14))) The culture vessel according to (((13))),
   in which the culture surface is, at least in part, offset outward at equal intervals from the distal end of the access instrument.
(((15))) The culture vessel according to (((13))) or (((14))),
   in which, in a state in which the outer peripheral surface is in contact with the inner peripheral surface, a minimum distance between an edge of the culture surface and the distal end of the access instrument is more than 0.2% and less than 20% with respect to a maximum dimension between the edges.
(((16))) The culture vessel according to any one of (((13))) to (((15))),
   in which contact portions with which the outer peripheral surface is configured to come into contact are formed at a plurality of locations on the inner peripheral surface, and each of the contact portions guides the distal end to a different position on the culture surface.
(((17))) The culture vessel according to any one of (((13))) to (((16))),
   in which a shape of the culture surface is non-circular.
(((18))) The culture vessel according to any one of (((13))) to (((18))),
   in which a cross-sectional area of a portion surrounded by the inner peripheral surface gradually decreases from the opening at the upper end portion toward the culture surface.
(((19))) A manufacturing method of a culture vessel, the manufacturing method comprising:
   a step of disposing, on a lower end surface of a main body portion that is formed in a tubular shape and that has openings at an upper end portion and a lower end portion, a bottom member that is formed with a culture surface on which cells are culturable and that is formed of a degradable material, in a state in which the opening is covered; and
   a step of heating the bottom member with a heat source that is disposed with a buffer member interposed between the bottom member and the heat source, and thermally fusion-welding the bottom member to the lower end surface,
   in which the buffer member has non-adhesiveness such that the bottom member does not adhere to the buffer member at a melting temperature of the bottom member.
(((20))) The manufacturing method of a culture vessel according to (((19))),
   in which the buffer member includes a fluororesin.

### <Supplementary Note 2>

(((1))) A culture vessel comprising:
   a main body portion that is formed in a tubular shape and that has an opening at an upper end portion,
   in which a lower end portion of the main body portion is provided with a bottom member that is formed with a culture surface on which cells are culturable, or is formed with an opening that is coverable by the bottom member that is formed with the culture surface on which cells are culturable, and
   an outer peripheral surface of an access instrument for accessing the culture surface, the access instrument being inserted from the opening at the upper end portion, comes into contact with at least a part of an inner peripheral surface of the main body portion so as to guide a distal end of the access instrument to a predetermined position on the culture surface.
(((2))) The culture vessel according to (((1))),
   in which the culture surface is, at least in part, offset outward at equal intervals from the distal end of the access instrument.
(((3))) The culture vessel according to (((1))) or (((2))),
   in which, in a state in which the outer peripheral surface is in contact with the inner peripheral surface, a minimum distance between an edge of the culture surface and the distal end of the access instrument is more than 0.2% and less than 20% with respect to a maximum dimension between the edges.
(((4))) The culture vessel according to any one of (((1))) to (((3))),
   in which contact portions with which the outer peripheral surface is configured to come into contact are formed at a plurality of locations on the inner peripheral surface, and each of the contact portions guides the distal end to a different position on the culture surface.
(((5))) The culture vessel according to any one of (((1))) to (((4))),
   in which a shape of the culture surface is non-circular.
(((6))) The culture vessel according to any one of (((1))) to (((5))),
   in which a cross-sectional area of a portion surrounded by the inner peripheral surface gradually decreases from the opening at the upper end portion toward the culture surface.
(((7))) The culture vessel according to any one of (((1))) to (((6))),
   in which the bottom member is formed of a degradable material.
(((8))) The culture vessel according to (((7))),
   in which the bottom member is a material that is degraded by biodegradation.
(((9))) The culture vessel according to any one of (((1))) to (((8))),
   in which the bottom member is a porous body.
(((10))) The culture vessel according to any one of (((1))) to (((9))),
   in which the bottom member is formed of a fibrous material.
(((11))) A culture mechanism comprising:
   a culture vessel including a main body portion that is formed in a tubular shape and that has an opening at an upper end portion, and a bottom member that is disposed at a lower end portion of the main body portion, and that is formed with a culture surface on which cells are culturable; and
   an access instrument that is insertable from the opening at the upper end portion, an outer peripheral surface of the access instrument of which comes into contact with at least a part of an inner peripheral surface of the culture vessel, a distal end of which is guided to a predetermined position on the culture surface.

The disclosure of Japanese Patent Application No. 2023-206456 filed on December 6, 2023 and the disclosure of Japanese Patent Application No. 2023-207161 filed on December 7, 2023 are incorporated herein by reference in their entirety. All documents, patent applications, and technical standards described in the present specification are herein incorporated by reference to the same extent that each individual document, patent application, or technical standard was specifically and individually indicated to be incorporated by reference.

## Claims

1. A culture vessel comprising:
a main body portion that is formed in a tubular shape and that has an opening at an upper end portion; and
a bottom member that is fixed to a lower end portion of the main body portion to constitute a bottom of the main body portion, and that is formed with a culture surface on which cells are culturable,
wherein the bottom member is formed of a degradable material.

2. The culture vessel according to claim 1,
wherein the bottom member is adhered to a lower end surface of the main body portion.

3. The culture vessel according to claim 2,
wherein the bottom member is thermally fusion-welded to the lower end surface.

4. The culture vessel according to claim 3,
wherein the bottom member is thermally fusion-welded to an outer portion of the lower end surface from a position offset outward from the culture surface.

5. The culture vessel according to claim 3,
wherein a surface free energy of the main body portion is -10 mJ/m² or more and +10 mJ/m² or less with respect to a surface free energy of the bottom member.

6. The culture vessel according to claim 1, further comprising:
a support member that includes
a support portion having higher rigidity than the bottom member, and
a holding portion that holds the support portion below the bottom member.

7. The culture vessel according to claim 6,
wherein the holding portion is fitted to the main body portion to hold the support portion.

8. The culture vessel according to claim 6,
wherein the holding portion is a pedestal that supports the support portion from below.

9. The culture vessel according to claim 1,
wherein the bottom member is a porous body.

10. The culture vessel according to claim 1,
wherein the bottom member is formed of a fibrous material.

11. The culture vessel according to claim 9 or 10, further comprising:
a support member that includes
a support portion having higher rigidity than the bottom member, and
a holding portion that holds the support portion below the bottom member,
wherein the support portion is formed of a porous body.

12. The culture vessel according to claim 1,
wherein the bottom member is a material that is degraded by biodegradation.

13. The culture vessel according to claim 1,
wherein an outer peripheral surface of an access instrument for accessing the culture surface, the access instrument being inserted from the opening at the upper end portion, comes into contact with at least a part of an inner peripheral surface of the main body portion so as to guide a distal end of the access instrument to a predetermined position on the culture surface.

14. The culture vessel according to claim 13,
wherein the culture surface is, at least in part, offset outward at equal intervals from the distal end of the access instrument.

15. The culture vessel according to claim 13,
wherein, in a state in which the outer peripheral surface is in contact with the inner peripheral surface, a minimum distance between an edge of the culture surface and the distal end of the access instrument is more than 0.2% and less than 20% with respect to a maximum dimension between the edges.

16. The culture vessel according to claim 13,
wherein contact portions with which the outer peripheral surface is configured to come into contact are formed at a plurality of locations on the inner peripheral surface, and each of the contact portions guides the distal end to a different position on the culture surface.

17. The culture vessel according to claim 13,
wherein a shape of the culture surface is non-circular.

18. The culture vessel according to claim 13,
wherein a cross-sectional area of a portion surrounded by the inner peripheral surface gradually decreases from the opening at the upper end portion toward the culture surface.

19. A manufacturing method of a culture vessel, the manufacturing method comprising:
a step of disposing, on a lower end surface of a main body portion that is formed in a tubular shape and that has openings at an upper end portion and a lower end portion, a bottom member that is formed with a culture surface on which cells are culturable and that is formed of a degradable material, in a state in which the opening is covered; and
a step of heating the bottom member with a heat source that is disposed with a buffer member interposed between the bottom member and the heat source, and thermally fusion-welding the bottom member to the lower end surface,
wherein the buffer member has non-adhesiveness such that the bottom member does not adhere to the buffer member at a melting temperature of the bottom member.

20. The manufacturing method of a culture vessel according to claim 19,
wherein the buffer member includes a fluororesin.
